(19) Europäisches Patentamt
European Patent Office
Office européen des brevets

(11) Publication number: 0 048 174
B1

(12) EUROPEAN PATENT SPECIFICATION

(45) Date of publication of patent specification: 22.02.84

(21) Application number: 81304254.6

(22) Date of filing: 16.09.81

(51) Int. Cl.³: C 07 C 69/15, C 07 C 67/36, C 07 C 67/37, B 01 J 27/08, B 01 J 31/08

(54) **Process for producing vinyl acetate.**

(30) Priority: 16.09.80 JP 128405/80

(43) Date of publication of application: 24.03.82 Bulletin 82/12

(45) Publication of the grant of the patent: 22.02.84 Bulletin 84/8

(84) Designated Contracting States: DE FR GB IT

(56) References cited:
EP - A - 0 025 702
DE - A - 1 966 695
DE - A - 2 610 035
DE - A - 2 842 267
DE - A - 2 856 791
US - A - 4 102 920
US - A - 4 102 921

(73) Proprietor: MITSUBISHI GAS CHEMICAL COMPANY, INC.
5-2, Marunouchi 2-chome Chiyoda-Ku
Tokyo (JP)

(72) Inventor: Isshiki, Tomiya
14-11, Umegaoka 2-chome
Setagaya-ku Tokyo (JP)
Inventor: Kijima, Yasuhiko
641, Koda
Matsudo-sho Chiba-ken (JP)
Inventor: Ito, Akira
15-6, Nishikubo-cho Tokiwadaira
Matsudo-shi Chiba-ken (JP)
Inventor: Miyauchi, Yuh
14-6, Jinyamae Tokiwadaira
Matsudo-shi Chiba-ken (JP)
Inventor: Kondo, Takao
61-6. Kikunodai 2-chome
Chofu-shi Tokyo (JP)
Inventor: Watanabe, Takayuki
6-1, Kanamachi 1-chome
Katsushika-ku Tokyo (JP)

(74) Representative: Cropp, John Anthony David et al,
MATHYS & SQUIRE 10 Fleet Street
London, EC4Y 1AY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 048 174 B1

# Process for producing vinyl acetate

This invention relates to a process for producing vinyl acetate which comprises reaction of methyl acetate and/or dimethyl ether, carbon monoxide and hydrogen.

Vinyl acetate has been produced from acetylene in the past. Recently vinyl acetate has also been produced from ethylene. In case of producing vinyl acetate from ethylene, palladium chloride or palladium chloride and sodium acetate has been used as a catalyst.

In addition, process for producing vinyl acetate which comprises producing ethylidene diacetate (1,1-diacetoxy ethane) from acetaldehyde and acetic anhydride and causing thermal decomposition of the ethylidene diacetate to form vinyl acetate, and process for producing vinyl acetate and acetic acid from acetaldehyde and acetic anhydride directly have been proposed (refer to Hydrocarbon Process, *44* (11) 287 (1965), British Patent No. 1,112,555 and U.S. Patent Nos. 2,021,698; 2,425,389 and 2,860,159).

European patent publication 0025702 describes a process for producing ethylidene diacetate by reacting methyl acetate or dimethyl ether, carbon monoxide and hydrogen in the presence of a catalyst comprising (a) as a main component at least one material selected from nickel, nickel compounds, cobalt and cobalt compounds and (b) as a secondary component at least one material selected from iodine, iodides, bromine and bromides under substantially anhydrous conditions.

German Offenlegungsschrift 2610035 describes a process for the production of ethylidene diacetate by the reaction of methyl acetate and/or dimethyl ether with carbon monoxide and hydrogen in the presence of a catalyst system comprising a Group VIII metal such as palladium, rhodium, iridium, platinum, ruthenium or osmium together with a bromide or iodide.

European patent publication 0035860 describes a process for the production of ethylidene diacetate and/or acetaldehyde by the hydrocarbonylation of methyl acetate or dimethyl ether with carbon monoxide and hydrogen in the presence of a catalyst comprising supported palladium and an iodide and/or bromide.

The present inventors carried out research to find a process for effectively synthesizing vinyl acetate from methyl acetate and/or dimethyl ether. As a result, we found a process for producing vinyl acetate in one stage by reacting methyl acetate or dimethyl ether, carbon monoxide and hydrogen.

According to the present invention, there is provided a process for producing vinyl acetate characterised in that it comprises reacting one or both of methyl acetate and dimethyl ether with hydrogen and carbon monoxide in the presence of a catalyst comprising

(a) at least one material selected from the group consisting of metals belonging to Group VIII of the Periodic Table, compounds of the metals and mixtures thereof and

(b) at least one iodide,

while maintaining the concentrations of the resulting vinyl acetate and acetic acid in the reaction system at less than 25% and less than 50% by weight, respectively, by removing vinyl acetate and acetic acid from the reaction system.

Figs. 1—2 show flow sheets of the apparatus for carrying out this invention.

The mechanism of the reaction of methyl acetate or dimethyl ether, carbon monoxide and hydrogen in this invention is not entirely clear. However, it is believed that the reaction is expressed by the following equations:

(1) When methyl acetate is used as a raw material

$$2CH_3COOCH_3+2CO+H_2 \rightarrow CH_2{=}CHOCOCH_3+2CH_3COOH$$

(2) When dimethyl ether is used as a raw material

$$2CH_3OCH_3+4CO+H_2 \rightarrow CH_2{=}CHOCOCH_3+2CH_3COOH$$

The above reaction proceeds in a good state when a catalyst comprising

(a) at least one material selected from the group consisting of metals belonging to Group VIII of the Periodic Table, compounds of the metals and mixtures thereof and

(b) at least one iodide is used.

The metals belonging to Group VIII of the Periodic Table can be used in any form of zero valence to higher valence state. For example, they can be used as metal itself or Raney metals, or finely divided particles of the metals, or as metal compounds, such as carbonates, oxides, peroxides, hydroxide, nitrates, sulfates, phosphates, halides, cyanides, thiocyanides, sulfonates, $C_1$—$C_5$ alkoxides, such as methoxides and ethoxides, phenoxide, carboxylates derived from $C_1$—$C_{20}$ alkanoic acids, oxyhalides, hydrides, carbonyls, nitrites, sulfites, phosphites, acetylacetonates and sulfides of metals or metal compounds coordinated with ammonia, cyanide, amines or amino acids.

The components (a) include, for example,

metallic Pd, $PdX_2$, $PdX_2 \cdot 2H_2O$, $PdX_2 \cdot 2NH_3$, $Pd(CN)_2$, $Pd_2H$, $Pd(OH)_2$, $Pd(OH)_2 \cdot 2NH_3$, $Pd(NO_3)_2$,

$Pd_2O$, PdO, $PdO_2$, $PdSO_4 \cdot 2H_2O$, $Pd_2S$, $[Pd(PPh_3)_2]Cl_2$, PdS, $PdS_2$, $Pd_3(PO_4)_2$, $Na_2PdX_4$, $Pd[n\text{-}C_4H_9)_3P](CO)Cl_2$, $K_2PdX_4$, $Li_2PdX_4$, $Pd(OAc)_2$, $Pd(AcAc)_2$, $PdX_2(PhCN)_2$, $Pd(SCN)_2$, $Pd(NC)_2$, palladium benzene sulfonate, metallic Rh, $RhX_3$, $RhX_3 \cdot 3H_2O$, $Rh(OH)_3$, $Rh(NO_3)_3 \cdot 2H_2O$, RhO, $RhO_2$, $RhO_3$, $Rh_2(SO_4)_3 \cdot 6H_2O$, RhS, $[Rh(AcO)_2]_2$, $Rh_2(CO)_3$, $Rh_6(CO)_{16}$, $RhCl(PPh_3)_3$, $[RhX(CO)_2]_2$, $Rh(AcAc)_3$, $Rh(SCK)_3$, $Rh(PPh_3)_2(CO)Cl_2$, $Rh(OPh)_3$, metallic Ir, $IrX_3$, $IrX_3 \cdot 3H_2O$, $IrO_2$, $IrO_2 \cdot 2H_2O$, $Ir_2O_3 \cdot 3H_2O$, IrS, $Ir_2(CO)_8$, metallic Pt, $H_2PtX_6$, $PtX_2$, $PtX_4 \cdot Pt(OH)_2$, $Pt(OH)_4$, $PtO_2$, PtO, $Pt(PPh_3)_2(SnCl_3)_2$, $Pt_3O_4$, $Pt(CO)X_2$, PtS, $Pt_2S_3$, $Pt(CK)_2$, metallic Ru, $RuX_2$, $RuX_3$, $RuX_4$, $Ru(OH)_3$, $RuO_2$, $Ru_2O_3$, $Ru(NO_3)_3 \cdot 6H_2O$, $Ru(CO)_2I_2$, $Ru(CO)_{12}$, metallic Os, $OsX_2$, $OsX_3$, $Os(CO)_4X_2$, $Os_3(CO)_{12}$, $Os(CO)_5$, metallic Fe, $FeS_2$, $FeX_2$, $Fe(NO_3)_2 \cdot 6H_2O$, $FeSO_4 \cdot 5H_2O$, $Fe(H_2PO_2)_3$, $FeSO_3$, $FeS_2O_3 \cdot 5H_2O$, $Fe(NO_2)_2$, metallic Ni, $NiX_2$, $NiX_2 \cdot 3H_2O$, NiO, $Ni_2O_3$, $Nio(CO)_4$, $NiCO_3$, $NiSO_4$, NiS, $Ni(CN)_2$, metallic Co, $Co_3O_4$, $CoX_3$, $Co(OAc)_2 \cdot 4H_2O$, $CoCO_3$, $Co_2(SO_4)_3$, $CoSO_4$,

wherein X is F, Cl, Br or I; Ph is phenyl group; AcO is acetoxy group and AcAc is acetylacetonate group.

The metal-polymer complexes in which a metal belonging to Group VIII of the Periodic Table is bonded to for example, silica, polyvinyl chloride, or polystyrene-divinylbenzene substrate croslinked by phosphine, silyl, an amine, pyridine or sulfido bond can be used as component (a).

The metal-polymer complexes are disclosed in CHEMTECH, 1973 Pages 560—566 and CHEMTECH, 1975 pages 117—122.

Of these metals and metal compounds, palladium, rhodium, platinum, ruthenium, osmium, cobalt and nickel and compounds of the metals may be used; palladium, rhodium, platinum, ruthenium, cobalt and nickel and compounds of the metals are preferable; and palladium, rhodium, cobalt and nickel and compounds of the metals are more preferable; and palladium, rhodium and compounds of the metals are most preferable. Of course, mixtures of metals belonging to Group VIII of the Periodic Table or compounds of these metals can also be used as component (a).

Component (a) constituting the catalyst may be soluble in a reaction solution. In this case a catalytic reaction is effected in a homogeneous system. The component (a) may also be insoluble in the reaction solution or partially soluble in the solution. In this case the reaction is effected in a heterogeneous system. The heterogeneous system catalysts include, for example, a metal itself, compounds of the metals or a metal supported on a carrier.

A metal can be supported on a carrier by coprecipitation, impregnation, blending, adsorption, and ion exchange and the like.

One of the processes for supporting a metal on a carrier is as follows: A carrier is impregnated with a solution of a metal belonging to Group VIII of the Periodic Table or a compound of the metal. The metal or the metal compound on the carrier is reduced with formalin, hydrogen, sodium formate, carbon monoxide, sodium borohydride, lithium aluminum hydride, or hydrazine, and then dried. Of course, the component (a) may be supported on a carrier by other methods.

Examples of the carriers include carbon, graphite, bone black, alumina, silica, silica alumina, barium sulfate, natural or synthetic zeolite, spinel, magnesia adhered alumina, thoria, titanium oxide, zirconium oxide, thorium oxide, lanthanum oxide, cerium oxide, zinc oxide, tantalum, clay, diatomaceous earth, Celite (trade name of product being commercially available from Johns-Manville Co.), asbestos, pumice stone, bauxite, terra abla, natural and treated terra abla, such as Super-Filtrol, silicon carbide, molecular sieves, ceramic honeycomb, boria, cements, alundum, corundum, brick, talc, gypsum and the like. Carbon, graphite, bone black, alumina, silica, silica alumina, barium sulfate, zeolite, spinel and magnesia adhered alumina are preferred. The carriers may be in the form of particles having uniform or ununiform size. The carriers may be in the form of molded articles, extruded articles, ceramic bars, balls, fragments, tiles and the like.

Heterogeneous system catalysts are preferred to homogeneous system catalysts, because separation and purification of the product, or separation or recovery of the catalyst are easy in a heterogeneous system.

Use of components (b) is critical with components (a). In general, the iodide or iodides may be added to the reaction medium in the form of an alkyl iodide, such as methyl iodide, an acid iodide, such as acetyl iodide, or hydrogen iodide. Materials which can convert to an alkyl iodide, an acid iodide or hydrogen iodide can be used as the iodide. Examples of the materials which convert to an alkyl iodide,

an acid iodide or hydrogen iodide by reacting with components in the reaction medium include inorganic iodides, such as alkali metal iodides, such as lithium iodide, sodium iodide, or potassium iodide; alkaline earth metal iodides, such as calcium iodide or magnesium iodide; metal iodides, such as aluminium iodide, zinc iodide, copper iodide, lanthanum iodides, or cerium iodides; or iodine.

Methyl iodide is more preferable, from the viewpoint of corrosion-resistance of reactor and separation of the reaction product from the reaction mixture and purification of the reaction product.

While the hydrocarbonylation reaction of methyl acetate or dimethyl ether of this invention proceeds effectively in the presence of the catalyst containing the above mentioned component (a) and the above mentioned component (b), promoters selected from compounds of nitrogen group elements, or metals or metal compounds may be used with the catalyst in order to increase reaction rate and to enhance selectivity to the object product. Preferable compounds of nitrogen group elements include organic compounds containing nitrogen, phosphorus, antimony or arsenic, ammonia or ammonium salts.

Examples of the compounds are shown in the following list which, however, is not exhaustive.

(I) trivalent compounds of nitrogen group elements

(A) Compounds represented by the formula

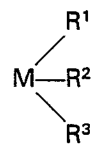

wherein M is N, P, Sb or As.

(a) compounds wherein $R^1$, $R^2$ and $R^3$ may be the same or different, and are independently hydrogen, saturated alkyl having 1—10 carbon atoms, saturated cycloalkyl having 3—10 carbon atoms, or aryl having 6—10 carbon atoms.

*M is N*

The compounds include, for example, ammonia, and amines, such as monomethyl amine, dimethyl amine, trimethyl amine, monoethylamine, diethyl amine, triethyl amine, dimethyl ethyl amine, tri-n-propyl amine, tri-isopropyl amine, tri-n-butyl amine, tri-tert.-butyl amine, aniline, dimethyl aniline, diethyl aniline, dimethylbenzyl amine, toluidine, triphenyl amine, cyclohexyl amine and the like.

*M is P*

The compounds include, for example, phosphines, such as tri-n-propyl phosphine, tri-iso-propyl phosphine, tri-n-butyl phosphine, tri-tert.-butyl phosphine, tricyclohexyl phosphine, triphenyl phosphine and the like.

*M is Sb*

The compounds include, for example, stibines, such as tri-iso-propyl stibine, ethyl-di-iso-propyl stibine, triphenyl stibine, tri(o-tolyl)stibine, phenyl diamyl stibine and the like.

*M is As*

The compounds include, for example, arsines, such as trimethyl arsine, triethyl arsine, tri-iso-propyl arsine, tri-n-propyl arsine, tricyclohexyl arsine, phenyl di-iso-propyl arsine, diphenyl arsine and the like.

(b) wherein $R^1$ is hydrogen, alkyl having 1—10 carbon atoms, cycloalkyl having 3—10 carbon atoms, or aryl and $R^2$ and $R^3$ are taken together and represents methylene or polymethylene having 2—5 carbon atoms; the compounds include, for example, pyrrolidine, N-methyl pyrrolidine, piperidine or N-phenyl piperidine:

(c) wherein $R^1$ and $R^2$ may be the same or different and independently represent hydrogen, alkyl having 1—10 carbon atoms, cycloalkyl having 3—10 carbon atoms, or aryl and having 6—10 carbon atoms and $R^3$ is aliphatic saturated acyl, or $R^1$ is hydrogen, alkyl having 1—10 carbon atoms, cycloalkyl having 3—10 carbon atoms or aryl having 6—10 carbon atoms, and $R^2$ and $R^3$ are taken together and represent lactam ring; the compounds include, for example carboxylamides such as acetamide, N,N-dimethylacetamide, acetanilide, N-methyl-N-phenylacetamide, and lactams, such as N-methylpyrrolidinone:

(d) wherein at least one of $R^1$, $R^2$ and $R^3$ is carboxymethyl and the remainder is hydrogen, alkyl having 1—10 carbon atoms, cycloalkyl having 3—10 carbon atoms or aryl having 6—10 carbon atoms; the compounds include, for example carboxylic acid derivatives, such as N,N-dimethyl glycine, N,N-diethyl glycine, iminodiacetic acid, N-methyl iminodiacetic acid, or nitrilotriacetic acid:

Organic compounds represented by the formula $N{\equiv}C{-}R$ wherein R represents an alkyl having 1 to 10 carbon atoms, a cycloalkyl having 3—10 carbon atoms or aryl; the compounds include for example nitriles, such as acetonitrile, propionitrile, or benzonitrile.

Organic nitrogen group compounds represented by the formula

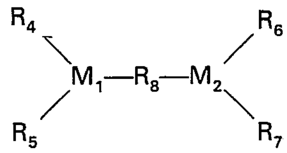

(a) wherein $R^4$, $R^5$, $R^6$ and $R^7$ may be the same or different and independently hydrogen, alkyl having 1—10 carbon atoms, cycloalkyl having 3—10 carbon atoms, or aryl and $R^8$ is methylene group or polymethylene group having 2—10 carbon atoms, phenylene, or carbonyl group

*$M^1$ and $M^2$ are N, P, Sb or As*

The compounds include, for example, ethylene bis(diphenylphosphine), phenylene bis(dimethylphosphine), bis(diphenylarsino)ethane, bis(di-iso-propylarsino)hexane, bis(diethylstibino)-pentane, N,N,N',N'-tetramethylethylenediamine, N,N,N',N'-tetraethylethylenediamine, N,N,N',N'-tetra-n-propylethylenediamine, N,N,N',N'-tetramethylmethylenediamine, N,N,N',N'-tetramethyl urea, N-methyl-2-pyrrodinone and triethylenediamine, (b) $R^4$ and $R^6$ may be the same or different and independently represent hydrogen, alkyl having 1—10 carbon atoms, cycloalkyl having 3—10 carbon atoms or aryl and $R^5$ and $R^7$ are taken together and represent methylene or polymethylene having 2—5 carbon atoms and $R^8$ is methylene or polymethylene having 2—5 carbon atoms; the compounds include, for example, heterocyclic compounds, such as piperazine, N-methyl-piperazine, N-ethyl-piperazine, or 2-methyl-N,N'-dimethylpiperazine.

(c) other compounds include, for example, tris(diethylaminomethyl)stibine, 2,5-dicarboxy-piperazine, cyclohexane-1,2-diamine-N,N,N',N'-tetraacetic acid or salts thereof, tetramethylester thereof, ethylenediaminetetraacetic acid, or its salt or tetramethylester thereof, 1,4-azabicyclo[2,2,2]octane, methyl substituted , 1,4-diazabicyclo[2,2,2]octane, adiponitrile or N-methylmorpholine.

(II) Hetero cyclic compounds include, for example, pyridines, such as pyridine; $\alpha$-picoline, $\beta$-picoline, 2-ethylpyridine, 3-ethylpyridine, 4-ethylpyridine, 2-propylpyridine, 4-propylpyridine, 4-butylpyridine, 4-isobutylpyridine, 4-tert.-butylpyridine, 2,6-lutidine, 2,4-lutidine, 2,5-lutidine, 3,4-lutidine, 3,5-lutidine, 2,4,6-collidine, 2-methyl-4-ethylpyridine, 2-methyl-5-ethylpyridine, 3-methyl-4-ethylpyridine, 3-ethyl-4-methylpyridine, 3,4-diethylpyridine, 3,5-diethylpyridine, 2-methyl-5-butylpyridine, 4-pentylpyridine, 4-(5-nonyl)-pyridine, 2,6-dipropylpyridine, 2-methyl-3-ethyl-6-propylpyridine, 2,6-diethylpyridine, 2,6-dipropylpyridine, 2,6-dibutylpyridine, 2,6-di-tert.-butylpyridine, functional group-containing pyridines, such as 2-cyanopyridine, 3-cyanopyridine, 4-cyanopyridine, 2,6-dicyanopyridine, 3,5-dicyanopyridine, 2-cyano-6-methylpyridine, 3-cyano-5-methylpyridine, picolinic amide, nicotinic amide, isonicotinic amide, picolinic acid, nicotinic acid, isonicotinic acid, dipicolinic acid, dinicotinic acid, cinchomethorinic acid, 5-butylpicolinic acid, alkyl esters of nicotinic acid, 2-aminopyridine, 3-aminopyridine, 4-aminopyridine, 2,3-diaminopyridine, 2,5-diaminopyridine, 2,6-diaminopyridine, 2,3,6-triaminopyridine, 2-amino-3-methylpyridine, 2-amino-4-methylpyridine, 2-amino-5-methylpyridine, 2-amino-6-methylpyridine, 2-amino-4-ethylpyridine, 2-amino-4-propylpyridine, 2-amino-4-(5-nonyl)pyridine, 2-amino-4,6-dimethylpyridine, 2,6-diamino-4-methylpyridine, 2,2'-dipyridylamine, 4-dimethylaminopyridine, 2-hydroxypyridine, 3-hydroxypyridine, 4-hydroxypyridine, 2,6-dihydroxypyridine, 3-hydroxy-6-methylpyridine, 2-chloropyridine, 2,6-dichloropyridine, 4-chloropyridine, 2-amino-5-chloropyridine, 2-amino-3,5-dichloropyridine, 2-methyl-3,5-dichloro-6-methylpyridine, 2-amino-5-chloro-3-methylpyridine, 2-amino-3,5-dichloro-4-methylpyridine, 2-amino-3,5-dichloro-4,6-dimethylpyridine, 4-amino-3,5-dichloropyridine, pyridine-N-oxide, $\beta$-picoline-N-oxide, $\alpha$-picoline-N-oxide, $\gamma$-picoline-N-oxide, 2,6-lutidine-N-oxide, 3,5-lutidine-N-oxide, 4-phenylpropylpyridine-N-oxide, 1,3-di-(4-pyridyl)-propane-di-N-oxide, 4-(5-nonyl)-pyridine-N-oxide, 2-chloropyridine-N-oxide, 4-cyanopyridine-N-oxide, 2-pyridine-methanol, 3-pyridinemethanol, 4-pyridinemethanol, 2,6-pyridinemethanol, 2-pyridineethanol, 4-pyridineethanol, 3-picolylamine, 4-picolylamine, 2-methylaminoethylpyridine, 4-alkylaminoethylpyridines, 4-piperidinoethylpyridine, 4-(4-pipecolinoethyl)pyridine, and 4-morpholinoethylpyridine, pyridines containing heterocyclic or homocyclic group, such as 2-phenylpyridine, 4-phenylpyridine, 2-benzylpyridine, 4-benzylpyridine, 4-phenylpropylpyridine, 4,4'-dipyridyl, 4,4'-dimethyl-2,2'-dipyridyl, 1,3-di-(4-pyridyl)propane, 1,2-di-(4-pyridyl)-ethane, 1,2-di-(4-pyridyl)-ethylene, 1,2,3-tri-(4-pyridyl)propane, 2,4,6-tri-(4-pyridyl)-S-triazine, 2,4-di(4-pyridyl)-6-methyl-S-triazine, and 2,5-di-(4-pyridyl)-S-tetrazine, alkenylpyridines or polymer pyridines, such as 2-vinylpyridine, 4-vinylpyridine, 2-vinyl-6-methylpyridine, 2-vinyl-5-ethylpyridine, 4-butenyl pyridine, pyridine, 4-vinylpyridine homopolymer, 2-vinylpyridine homopolymer, 2-vinylpyridine-acrylonitrile copolymer, 4-vinylpyridine-acrylonitrile copolymer, 4-vinylpyridine-styrene copolymer, 4-vinylpyridine-divinylbenzene copolymer, 2-vinylpyridine-divinylbenzene copolymer, and 4-vinylpyridine homopolymer-N-oxide; pyrroles; pyrrolines; pyrinidines; pyrazines; pyrazoles; pyrazolines; pyridazines; imidazoles; 1,10-phenanthrolines, such as 1,10-phenanthroline 4-chloro-1,10-phenanthroline, and 5-(thiabentyl)-1,10-phenanthroline; quinolines, such as quinoline, 2-(dimethylamino)-6-methoxyquinoline, 8-hydroxyquinoline and 2-carboxyquinoline.

(III) Pentavalent nitrogen group compounds

The compounds include, for example ammonium acetate, ammonium propionate, and triphenylphosphineiminiumchloride.

Of these nitrogen group compounds, ammonia, ammonium salts and organic compounds containing nitrogen atom or phosphorus atom are preferable; organic compounds containing trivalent phosphorus atom or trivalent nitrogen atom are more preferable; and phosphine compounds containing trivalent phosphorus atom and heterocyclic compounds containing trivalent nitrogen atom, such as pyridines are most preferable.

Metals or metal compounds which can be used as promoters include, for example metals having atomic weight of at least 6 and belonging to Groups Ia, IIa, IIIa, IVa, Va, Ib, IIB, Vb, VIb and VIIb of the Periodic Table and compounds of these metals. Preferable metals or metal compounds include, for example, metals having atomic weight of less than 210 and belonging to said Groups and compounds of the metals.

Metals belonging to said Groups include lithium, sodium, potassium, rubidium, cesium, magnesium, calcium, strontium, barium, aluminum, tin, antimony, bismuth, zinc, cadmium, copper, manganese, chromium and vanadium; lithium, sodium, potassium, rubidium, cesium, magnesium, calcium, strontium, barium, aluminum, tin and antimony are preferable; and lithium, potassium, rubidium, cesium, aluminum, barium and antimony are more preferable; and lithium, potassium, barium and antimony are most preferable.

The inorganic promoters may be used in form of elements, for example finely divided particles of the metals; or the inorganic promoters may be used in form of cations, for example organic or inorganic compounds of the metals. Typical compounds of the metals include, for example oxides, hydroxides, halides, such as chlorides, fluorides bromides or iodides, oxyhalides, hydrides, carbonyls, alkoxides, sulfates, sulfites, nitrates, nitrites, phosphates, phosphites, aliphatic, alicyclic and aromatic carboxylates, such as acetates, butyrates, decanates, laurates, stearates, benzoates of the metals. Halides, such as chlorides, fluorides, bromides and iodides and carboxylates, such as acetate of the metals are more preferable. The inorganic promoters may be used for increasing selectivity to vinyl acetate. So, use of both nitrogen group compounds and inorganic promoters are particularly profitable.

These promoters may be supported on a porous inorganic material with a component (a) supported on a porous inorganic material. It is preferred that promoter be supported on a porous inorganic material with a palladium metal.

The amount of component (a) used in this invention varies with the type of the catalyst (whether it is homogeneous or heterogeneous) or in case of a heterogeneous catalyst, with the type of the catalyst bed (whether it is fluidized or fixed). However, in general, the amount of the component (a) employed may be in the range of from $1 \times 10^{-4}$ to 25 wt%, preferably in the range of from $5 \times 10^{-4}$ to 20 wt% more preferably from $1 \times 10^{-3}$ to 15 wt% and most preferably from $2.5 \times 10^{-3}$ to 10 wt% on the basis of weight of a reaction solution in terms of metal.

Amount of iodide employed as component (b) may be in the range of from $10^{-3}$ to 15 mol, preferably $10^{-2}$ — 5 mol and preferably $10^{-1}$ — 3 mol per 1 liter of a reaction solution in terms of halogen atom.

Amount of the promoter employed may be in the range of from $1 \times 10^{-6}$ to 10 mol, preferably $1 \times 10^{-4}$ to 5 mol and most preferably $1 \times 10^{-3}$ to 2.5 mol per 1 liter of a reaction solution.

In case of practising this invention, the reaction temperature is not critical. In general, the reaction temperature may be in the range of 20—500°C, preferably 80—350°C and most preferably 100—250°C.

The reaction pressure is kept high enough to keep the raw material(s), the solvent and the product in a liquid phase and to maintain appropriately partial pressure of hydrogen or carbon monoxide. The partial pressure of hydrogen or carbon monoxide may be in the range of 0.5—500 atm. preferably 1—400 atm. and more preferably 3—300 atm. However, partial pressure of hydrogen or carbon monoxide may be in the range of 0.05—1000 atm.

Dimethyl ether and methyl acetate which are used as raw materials in this invention may be prepared by known methods. For example, dimethyl ether may be produced by dehydration-dimerization reaction of methanol.

The methyl acetate may be produced by esterification of methanol and acetic acid. In this reaction acetic acid may be produced by reacting methanol with carbon monoxide (ref. Japan Patent Publication (laid open) Nos. 59211/1979, 63002/1979 and 66614/197). Acetic acid which is formed as a by-product in synthesis of vinyl acetate by the present invention may be used for synthesis of methyl acetate. Methyl acetate may also be produced by reacting methanol with synthesis gas. [Japanese Patent Publication for opposition No. 2525/1973, Patent Publication (laid open) Nos. 136110/1977 and 136111/1977]. Vinyl acetate is converted to polyvinyl acetate. Methyl acetate formed as a by-product when the polyvinyl acetate is converted to polyvinyl alcohol may be used as a raw material. Methanol, dimethyl ether, acetic acid, acetaldehyde, dimethyl acetal and iodides, such as methyl iodide may be incorporated in the methyl acetate employed as a raw material of this invention. However, incorporation of these compounds in methyl acetate is permitted as long as an overall balance is

obtained.

Methyl acetate produced from acetic acid formed as a by-product in production of PVA from vinyl acetate produced in the present invention may be used as a starting material of the present invention.

Stoichiometric amounts of the raw material gas depend on which one of methyl acetate, dimethyl ether and mixture thereof is used. Therefore, stoichiometric amounts of carbon monoxide and hydrogen may vary from molar ratio of CO to $H_2$ of 2:1 to molar ratio of CO to $H_2$ of 4:1. It is not necessarily critical to use CO and $H_2$ in stoichiometric amounts. In general, the molar ratio of CO to $H_2$ may be in the range of from 1:100 to 100:1, preferably 1:50 to 50:1 and more preferably 1:10 to 10:1. However, it is preferable that carbon monoxide and hydrogen be used in an approximately stoichiometric amount. So, preferably, the rato of carbon monoxide to hydrogen may be in the range of 0.5:1 to 5:1.

In the practice of this invention, carbon monoxide and hydrogen may be introduced into the reaction system as separate streams. Synthesis gas comprising carbon monoxide and hydrogen may be introduced into the reaction system. The raw material gas composed of carbon monoxide and hydrogen does not need to be highly pure and may contain carbon dioxide, methane, nitrogen, and rare gases. Extremely low concentration of either of carbon monoxide or hydrogen in the mixed gas is not desirable, because the reaction pressure must rise when using such gas.

In general, water may be necessarily incorporated into the reaction system. However, since commercially available raw material gas and methyl acetate or dimethyl ether contains a small amount of water, raw material gas and methyl acetate or dimethyl ether containing water of such low concentration are permitted in this invention. The presence of water of more than 10 mol % on the basis of a reacton solution is not preferable in this process, because such large amount of water causes to decompose the starting materials and the products.

In general, a water content of less than 5 mol % is preferable, and a water content of less than 3 mol % is more preferable. When raw materials contain a large amount of water, they should be dried before introducing them into the reaction system.

Since methyl acetate or dimethyl ether (as a starting material), vinyl acetate (object product) or acetic anhydride or ethylidene diacetate (intermediate) serves as a solvent for the reaction of this invention, another solvent is not necessarily used. Particularly, ethylidene diacetate and acetic anhydride are excellent solvents in this invention. However, use of a separate, solvent increases selectivity to vinyl acetate (object product).

Solvents which are usable for such purpose have dielectric constants of less than 18 at 25°C. Organic solvents having dielectric constants of less than 15 at 25°C are preferable; organic solvents having dielectric constants of less than 10 at 25°C are more preferable; and organic solvents having dielectric constants of less than 8 at 25°C are most preferable.

In general, the organic solvents include aliphatic hydrocarbons having 5—20 carbon atoms, aromatic hydrocarbons having 6—20 carbon atoms, organic acid esters having 3—20 carbon atoms, ethers having 4—20 carbon atoms, ketones having 3—20 carbon atoms, alcohols having 4—20 carbon atoms and halogenated hydrocarbons having 1—20 carbon atoms. Of these solvents, saturated aliphatic hydrocarbons, aromatic hydrocarbons and organic acid esters are preferable.

Examples of the organic solvents include aliphatic hydrocarbons, such as pentane, hexane, heptane, octane, nonane, decane, undecane, dodecane, tridecane, tetradecane, pentadecane, hexadecane, cyclopentane, cyclohexane, and their alkylated derivatives; aromatic hydrocarbons, such as benzene, toluene, o-, p- and m-xylenes, trimethyl benzenes, ethyl benzene, propyl benzene, naphthalene, biphenyl, diphenyl methane, and their alkylated derivatives; organic acid esters, such as methyl acetate, ethyl acetate, propyl acetate, butyl acetate, amyl acetate, hexyl acetate, cyclohexyl acetate, octyl acetate, phenyl acetate, tolyl acetate, benzyl acetate, butyl formate, amyl formate, methyl propionate, ethyl propionate, methyl butyrate, ethyl butyrate, methyl benzoate, ethyl benzoate, methyl toluylate, dimethyl adipate, ethylene glycol diacetate, ethylidene diacetate, diethylene glycol diacetate, propylene glycol diacetate, $\alpha$-valerolactone, and $\delta$-caprolactone; ethers, such as diethyl ether, dipropyl ether, methyl butyl ether, dibutyl ether, butyl ethyl ether, diamyl ether, diphenyl ether, anisole, tetrahydrofuran, 1,4-dioxane, butyl propyl ether, benzyl ethyl ether, phenetole, and dibenzyl ether; ketones, such as acetophenone, cyclohexanone, 3-pentanone and 4-methyl-2-pentanone; alcohols, such as 1-pentanol, 3-methyl-1-butanol, 3-methyl-2-propanol, 1-hexanol, 1-octanol, cyclohexanol, phenol and benzyl alcohol; halogenated hydrocarbons, such as methyl iodide, dichloromethane, chloroform, carbon tetrachloride, chlorobenzene, dichlorobenzenes and iodobenzene. Mixtures of the above solvents may also be used.

The amount of solvent employed is not critical. In general, it may be in the range of 0 to 95 wt%, preferably 2 to 90 wt%, preferably 5 to 90 wt% and most preferably 5 to 80 wt% on the basis of weight of reaction solution. It is preferable to select the kind and amount of solvent so that the dielectric constant of the reaction solution amounts to less than 19, preferably less than 18 and more preferably less than 17 at 25°C.

The present process may be carried out by batch, semi-continuous or continuous method. When a heterogeneous system catalyst is used, the reaction can be effected in a fluidized bed or a fixed bed.

In the present invention, the reaction solution comprises the catalyst methyl acetate or dimethyl ether (starting material), acetic anhydride (intermediate), ethylidene diacetate (intermediate), vinyl

acetate (object product) and by-products containing acetic acid. In accordance with the invention, the concentration of vinyl acetate in the reaction system is maintained at less than 25 wt% by removing (for example distilling) the resulting vinyl acetate from the reaction system. It is preferable to maintain the vinyl acetate concentration at less than 15 wt%; and it is more preferable to maintain the concentration at less than 10 wt%. Particularly, it is most preferable to maintain the concentration at less than 5 wt%. Such withdrawal of the vinyl acetate increases the yield.

Likewise, the concentration of acetic acid in the reaction system is maintained at less than 50 wt%, preferably less than 40 wt%, more preferably less than 30 wt% and most preferably less than 20 wt% by removing acetic acid from the reaction system.

Withdrawal of vinyl acetate and acetic acid from the reaction system may be carried out, for example, by distillation.

For better understanding of this invention, preferred embodiments of the invention are described hereunder by reference to the non-limiting accompanying drawings.

In Fig. 1, reaction zone 10 is composed of one or more pressure vessel of any type. Into the vessel are charged a suitable catalyst system typically consisting of methyl iodide and a metal belonging to Group VIII of the Periodic Table or a compound of the metal and other liquid solution. Raw material gas containing hydrogen and carbon monoxide is fed under pressure at the bottom of zone 10 through line 11. Methyl acetate is fed into zone 10 through line 12. The reaction mixture, namely materials containing the reaction products, unreacted raw materials and intermediates are introduced from zone 10 through line 13 to separating zone 20 consisting of one or more distilling unit, for example flashing means and/or fractionating columns. High boiling point components containing non-volatile catalyst components separated in zone 20 are recycled into reaction zone 10 through line 21. Low boiling point components containing acetaldehyde, methyl iodide, methyl acetate, vinyl acetate and acetic acid separated in zone 20 are discharged through line 22 and fed into separating zone 30 consisting of one or more distilling units. Acetaldehyde, methyl iodide and methyl acetate separated in zone 30 are recycled into reaction zone 10 through line 33. Vinyl acetate and acetic acid separated in zone 30 are discharged together from lines 31 or 32, or are discharged separately from lines 31 and 32.

In Fig. 2, reaction zone 100 is composed of one or more pressure vessels of any type. Into the vessel are charged a suitable catalyst system typically consisting of methyl iodide and a metal belonging to Group VIII of the Periodic Table or a compound of the metal and reaction solution. Raw material gas containing hydrogen and carbon monoxide is fed with recycling gas stream into bottom of zone 100 through line 101. Methyl acetate is fed into zone 100 through line 102. Reaction products and unreacted materials are withdrawn from zone 100 through line 105, and fed into separating zone 200 consisting of one or more distilling units, for example flashing means and/or fractionating columns. Gas containing hydrogen and carbon monoxide separated in zone 200 are recycled into zone 100 through lines 108 and 109. As occasion demands, part of the gas is discharged outside through line 110. Acetaldehyde, methyl iodide, methyl acetate, vinyl acetate and acetic acid are gas-liquid-separated by stripping and/or evaporation in zone 200. Part of acetic acid, part of acetic anhydride, and most of acetaldehyde, methyl iodide and methyl acetate are recycled into vessel 100 with gas containing hydrogen and carbon monoxide. Components containing as main components vinyl acetate and acetic acid gas-liquid-separated in zone 200 is discharged from zone 200 through line 107 and fed into separating zone 300. Low boiling point components comprising mainly methyl acetate and methyl iodide separated in zone 300 are recycled into zone 100 through line 304. High boiling point components comprising acetic anhydride and ethylidene diacetate are recycled from zone 300 to vessel 100 through line 304. Vinyl acetate (object product) and acetic acid (by-product) are discharged together from lines 301 or 302, or discharged separately from lines 301 and 302. As occasion demands, in order to prevent accumulation of polymers formed as by-products during the reaction and other high boiling point materials or in order to withdraw part of the catalyst, part of the reaction solution is discharged from zone 100 through line 104.

The following examples are given as illustrative embodiments of the invention and should not be construed as limiting its scope.

All parts and percents are on weight, unless otherwise specified.

### Example 1

This example was carried out by using the apparatus as shown in Fig. 1. Into pressure zone 10 was charged a mixture of 46.4% of methyl acetate, 11.1% of methyl iodide, 39.5% of ethylidene diacetate, 1.58% of 2,6-lutidine, 0.59% of palladium acetate and 0.83% of palladium (5%) supported on activated carbon, in a determined amount. Air in zone 10 was purged with mixed gas of carbon monoxide and hydrogen (2:1 by volume). The temperature of zone 10 was raised to 175°C. The mixed gas was fed from bottom of zone 10 under pressure to 100 Kg/cm$^2$G through line 11. The mixture was stirred for 4.3 hours while maintaining the pressure of 100 Kg/cm$^2$G by feeding the mixed gas into zone 10. Thereafter, movement of the solution started so that average residence time of the solution amounted to 3 hours, and at the same time methyl acetate was fed into zone 10 from line 12 at a rate of 9.01 g/hour, and the mixed gas was continuously fed into zone 10 through line 11 so as to maintain the pressure at 100 Kg/cm$^2$G. The reaction mixture withdrawn from line 13 was fed into separating

zone 20 and flash-distilled there to obtain low boiling point components containing 5.01% of vinyl acetate. The low boiling point components were fed into separating zone 30 through line 22. High boiling point components and the catalyst separated in zone 20 were recycled into zone 10 through line 21. The components charged into zone 30 were separated there. Low boiling point components mainly composed of methyl acetate and methyl iodide were recycled into zone 10 through line 33, and high boiling point components mainly composed of acetic anhydride and ethylidene diacetate were recycled into zone 10 through line 34. Vinyl acetate (object product) was discharged from zone 30 through line 32 at rate of 4.92 g/hour and acetic acid was discharged from zone 30 through line 31 at rate of 7.29 g/hour.

Example 2

This example was carried out by using the apparatus as shown in Fig. 2. Into pressure zone 100 was charged a mixture of 46.4% of methyl acetate, 11.1% of methyl iodide, 39.5% of ethylidene diacetate, 1.58% of 2,6-lutidine, 0.59% of palladium acetate and 0.83% of palladium (5%) supported on activated carbon, in a determined amount. Air in zone 10 was purged with mixed gas of carbon monoxide and hydrogen (2:1 by volume). The temperature of zone 100 was raised to 175°C. The mixed gas was fed from bottom of zone 100 under pressure to 100 $Kg/cm^2G$ through line 101. The mixture was stirred for 4.3 hours while maintaining the pressure of 100 $Kg/cm^2G$ by feeding the mixed gas into zone 100. Thereafter movement of the solution started so that average residence time of the solution amounted to 3 hours, and at the same time methyl acetate was fed into zone 100 from line 102 at a rate of 9.39 g/hour, and the mixed gas was continuously fed into zone 100 so as to maintain the pressure at 100 $Kg/cm^2G$. Gas was circulated through zone 100, line 105, separating zone 200 including distilling units, and lines 108, 109 and 103. Components containing 6.47% of vinyl acetate condensed at 100°C in zone 200 were fed into separating zone 300 including fractionating means through line 107. Low boiling point components mainly composed of methyl acetate and methyl iodide, which were separated in zone 300 were recycled into zone 100 through line 303, and high boiling point components mainly composed of acetic anhydride and ethylidene diacetate, which were separated in zone 300 were recycled into zone 100 through line 304. Vinyl acetate (object product) was discharged from zone 300 through line 302 at a rate of 5.22 g/hour. Acetic acid was discharged from zone 300 through line 301 at a rate of 7.52 g/hour.

The following Examples illustrate the variety and range of catalyst components, promoters and reaction conditions that may be employed.

Example 3

Into an autoclave were charged 105 g of methyl acetate, 39 g of methyl iodide, 1.09 g of 2,6-lutidine and 4.32 g of palladium (5%) supported on activated carbon (being commercially available from Nippon Engelhard). Mixed gas of carbon monoxide and hydrogen (2:1 by volume) was fed under pressure into the autoclave. The reaction was carried out at 175°C and 100 $Kg/cm^2G$ for 4 hours. After cooling the reaction mixture, analysis showed that it contained 0.351 g of vinyl acetate, 7.68 g of ethylidene diacetate, 0.203 g of acetic anhydride, 5.22 g of acetaldehyde and 2.265 g of ethyl acetate with considerable amount of acetic acid.

Examples 4—17

The procedures of Example 3 were repeated by using starting materials, catalysts, promoters and solvents and the reaction conditions. The results are shown in Table 1.

TABLE 1

| | | Ex, 4 | Ex. 5 | Ex. 6 |
|---|---|---|---|---|
| Methyl acetate (g) | | 105 | 105 | 120 |
| Catalyst | | methyl iodide (39 g), Pd (5%) supported on activated carbon** (4.32 g) | Same as the left | methyl iodide (30 g), palladium chloride (0.451 g) |
| Promoter | | 2,6-lutidine (1.09 g) lithium acetate (4.5 g) | potassium sulfate (4.5 g), 2,6-lutidine (1.09 g) | tri-n-butyl-phosphine (1.28 g) |
| Ratio of CO to $H_2$ (by volume) | | 2:1 | 2:1 | 2:1 |
| Solvent | | | | |
| Reaction Condition | Temperature (°C) | 175 | 175 | 180 |
| | Pressure (Kg/cm²G) | 100 | 100 | 100 |
| | Time | 4 hours | 4 hours | 3 hours |
| Components in reaction mixture | Vinyl acetate (g) | 0.562 | 0.372 | 0.211 |
| | EDA* (g) | 6.37 | 0.781 | 6.30 |
| | Acetic anhydride (g) | 0.250 | 3.76 | 4.15 |
| | Acetaldehyde (g) | 4.80 | 2.39 | |
| | Acetic acid | considerable amount | same as the left | same as the left |
| | Ethyl acetate (g) | 0.196 | | |

* ethylidene diacetate
** Nippon Engelhard.

TABLE 1 (Continued)

| | | Ex. 7 | Ex. 8 | Ex. 9 |
|---|---|---|---|---|
| Methyl acetate (g) | | 105 | 120 | 120 |
| Catalyst | | methyl iodide (39 g) palladium chloride (0.451 g) | $CH_3I$ (30 g), rhodium acetate (0.611 g), Pd (5%) supported on activated carbon** (4.5 g) | $CH_3I$ (30 g), rhodium chloride (1.92 g), Pd (5%) supported on activated carbon (4.5 g) |
| Promoter | | tri-n-butylphosphine (1.28 g), KCl (4.5 g) | lithium acetate (2.88 g) | triphenyl phosphine (9.56 g) |
| Ratio of CO to $H_2$ (by volume) | | 2:1 | 2:1 | 2:1 |
| Solvent | | | | |
| Reaction Condition | Temperature (°C) | 175 | 175 | 175 |
| | Pressure (Kg/cm²G) | 100 | 100 | 100 |
| | Time | 3 hours | 160 min. | 45 min. |
| Components in reaction mixture | Vinyl acetate (g) | 0.226 | 0.520 | 0.147 |
| | EDA* (g) | 0.733 | 73.7 | 53.1 |
| | Acetic anhydride (g) | 5.56 | 25.7 | 10.2 |
| | Acetaldehyde (g) | | | 0.544 |
| | Acetic acid | considerable amount | same as the left | same as the left |
| | Ethyl acetate (g) | | | 0.257 |

**Nippon Engelhard.

*ethylene diacetate.

TABLE 1 (Continued)

| | | Ex. 10 | Ex. 11 | Ex. 12 |
|---|---|---|---|---|
| Methyl acetate (g) | | 105 | 105 | 112 |
| Catalyst | | $CH_3I$ (39 g)<br>rhodium iodide (2.11 g) | $CH_3I$ (39 g)<br>rhodium iodide (2.11 g) | $CH_3I$ (37.7 g)<br>rhodium chloride (1.85 g) |
| Promoter | | 2,6-lutidine (12 g),<br>LiI (4.5 g) | 2,6-lutidine (1.09 g),<br>LiBr (4.5 g) | triphenyl phosphine (9.32 g),<br>$BaI_2.2H_2O$ (4.5 g) |
| Ratio of CO to $H_2$ (by volume) | | 2:1 | 2:1 | 2:1 |
| Solvent | | | | |
| Reaction Condition | Temperature (°C) | 175 | 175 | 150 |
| | Pressure ($Kg/cm^2G$) | 100 | 100 | 100 |
| | Time | 3 hours | 3 hours | 3 hours |
| Components in reaction mixture | Vinyl acetate (g) | 3.03 | 0.976 | 2.91 |
| | EDA* (g) | 46.7 | 46.2 | 53.7 |
| | Acetic anhydride (g) | 17.3 | 17.9 | 18.6 |
| | Acetaldehyde (g) | | | |
| | Acetic acid | same as the left | considerable amount | same as the left |
| | Ethyl acetate (g) | | | |

*ethylene diacetate.

TABLE 1 (Continued)

| | | Ex. 13 | Ex. 14 | Ex. 15 |
|---|---|---|---|---|
| Methyl acetate (g) | | 105 | 105 | 120 |
| Catalyst | | $CH_3I$ (39 g)<br>rhodium iodide (2.11 g) | $CH_3I$ (39 g)<br>rhodium iodide (2.11 g) | $CH_3I$ (32.4 g)<br>Ni powder (1.91 g)<br>Pd (5%) supported on activated carbon (4.80 g) |
| Promoter | | 2,4-lutidine (8.0 g)<br>antimony iodide (4.5 g) | 2,6-lutidine (12 g)<br>tin (IV) iodide (4.5 g) | 2,4-lutidine (10.5 g) |
| Ratio of CO to $H_2$ (by volume) | | 2:1 | 2:1 | 2:1 |
| Solvent | | | | |
| Reaction Condition | Temperature (°C) | 175 | 175 | 175 |
| | Pressure ($Kg/cm^2G$) | 100 | 100 | 100 |
| | Time | 3 hours | 3 hours | 3 hours |
| Components in reaction mixture | Vinyl acetate (g) | 2.14 | 1.51 | 0.480 |
| | EDA* (g) | 46.2 | 38.7 | 7.56 |
| | Acetic anhydride (g) | 19.9 | 14.9 | 29.8 |
| | Acetaldehyde (g) | | | |
| | Acetic acid | same as the left | considerable amount | same as the left |
| | Ethyl acetate (g) | | | |

*ethylidene diacetate.

TABLE 1 (Continued)

| | | Ex. 16 | Ex. 17 |
|---|---|---|---|
| Methyl acetate (g) | | 100 | 30 |
| Catalyst | | $CH_3I$ (30 g)<br>palladium acetate (0.316 g)<br>Pd (5%) supported on activated carbon (1.5 g) | $CH_3I$ (30 g)<br>Pd (5%) supported on an activated carbon (4.5 g) |
| Promoter | | 2,6-lutidine (1.13 g) | 2,6-lutidine (1.13 g) |
| Ratio of CO to $H_2$ (by volume) | | 2:1 | 2:1 |
| Solvent | | acetic anhydride (20 g) | ethylidene diacetate (90 g) |
| Reaction Condition | Temperature (°C) | 175 | 175 |
| | Pressure ($Kg/cm^2G$) | 100 | 100 |
| | Time | 4 hours | 4 hours |
| Components in reaction mixture | Vinyl acetate (g) | 0.501 | 2.91 |
| | EDA* (g) | 7.54 | 93.8 |
| | Acetic anhydride (g) | 4.72 | |
| | Acetaldehyde (g) | 5.37 | |
| | Acetic acid | same as the left | |
| | Ethyl acetate (g) | 0.0911 | |

*ethylidene diacetate.

Example 18

Into an autoclave were charged 120 g of methyl acetate, 30 g of methyl iodide, 3.40 g of 2,6-lutidine, 0.949 g of palladium acetate and 4.5 g of palladium (5%) supported on activated carbon (Nippon Engelhard). Mixed gas of carbon monoxide and hydrogen (9:1 by volume) was fed under pressure to pressure of 100 $Kg/cm^2$. The temperature was raised to 175°C. The reaction was carried out at 175°C for 4 hours while maintaining the pressure at 100 $Kg/cm^2$ by continuously feeding mixed gas of $CO:H_2$ (2:1 by volume) into the autoclave. The reaction mixture contained 0.238 g of vinyl acetate, 3.35 g of ethylidene diacetate, 2.11 g of acetic anhydride and 1.86 g of acetaldehyde with considerable amount of acetic acid.

Example 19

Into an autoclave were charged 88.8 g of methyl acetate, 72.0 g of ethylidene diacetate, 41.7 g of methyl iodide, 4.56 g of 2,4-lutidine, 1.25 g of nickel powder and 7.92 g of lithium acetate. Air in the autoclave was purged with carbon monoxide. The temperature was raised to 180°C with stirring. Carbon monoxide under pressure of 55 $Kg/cm^2$ and hydrogen under pressure of 10 $Kg/cm^2$ were fed into the autoclave so that total pressure of the autoclave amounted to 80 $Kg/cm^2$. Then the reaction was carried out at that temperature for 5 hours. During the reaction the pressure of the autoclave was maintained at 80 $Kg/cm^2$ by continuously feeding mixed gas of $CO:H_2$ (2:1 by volume) into the autoclave. After cooling the reaction mixture, analysis showed that it contained 4.90 g of vinyl acetate and 46.3 g of acetic anhydride with considerable amount of acetic acid and ethylidene diacetate.

Example 20

Into an autoclave 111 g of methyl acetate, 41.7 g of methyl iodide, 4.56 g of 2,4-lutidine, 1.25 g of nickel powder, 1.00 g of cobalt octacarbonyl and 7.92 g of lithium acetate. Air in the autoclave was purged with mixed gas of $CO:H_2$ (2:1 by volume). The temperature was raised to 180°C. The mixed gas was fed under pressure to pressure of 100 $Kg/cm^2$. The reaction was carried out at that temperature for 3 hours while maintaining the pressure at 100 $Kg/cm^2$ by continuously feeding the mixed gas into the autoclave. After cooling the reaction mixture, analysis showed that it contained 0.32 of vinyl acetate, 1.75 g of ethylidene diacetate and 32.1 g of acetic anhydride with considerable amount of acetic acid.

Example 21

Into an autoclave were charged 30 g of methyl iodide, 90 g of ethylidene diacetate, 1.13 g of 2,6-lutidine and 4.5 g of palladium (5%) supported on activated carbon. After air in the autoclave was purged with nitrogen, 30 g of dimethyl ether was charged into the autoclave. Mixed gas of $CO:H_2$ (2:1 by volume) was fed under pressure so pressure of the autoclave amounted to 200 $Kg/cm^2$; and the reaction was carried out at 175°C for 8 hours. The reaction mixture contained 0.15 g of vinyl acetate and 91.8 g of ethylidene diacetate.

**Claims**

1. A process for producing vinyl acetate characterised in that it comprises reacting one or both of methyl acetate and dimethyl ether with hydrogen and carbon monoxide in the presence of a catalyst comprising

(a) at least one material selected from the group consisting of metals belonging to Group VIII of the Periodic Table, compounds of the metals and mixtures thereof and

(b) at least one iodide, while maintaining the concentrations of the resulting vinyl acetate and acetic acid in the reaction system at less than 25% and less than 50% by weight, respectively, by removing vinyl acetate and acetic acid from the reaction system.

2. The process as defined in Claim 1 characterised in that catalyst component (a) is selected from palladium, rhodium, cobalt, nickel and compounds of these metals.

3. The process as defined in Claim 1 or Claim 2 characterised in that the iodide is selected from alkyl iodides, acid iodides, hydrogen iodide and mixtures thereof.

4. The process as defined in any one of Claims 1 to 3 characterised in that said reaction is carried out in the presence of said catalyst and together with at least one promoter, said promoter being selected from nitrogen group compounds, metals having an atomic weight of at least 6 and belonging to Groups Ia, IIa, IIIa, IVa, Va, Ib, IIb, Vb, VIb and VIIb of the Periodic Table and compounds of these metals.

5. The process as defined in Claim 4 characterised in that said promoter is a nitrogen group compound selected from organic compounds containing trivalent phosphorus and organic compounds containing trivalent nitrogen.

6. The process as defined in Claim 4 or Claim 5 characterised in that the nitrogen group compound is a heterocyclic compound.

7. The process as defined in Claim 4 characterised in that said promoter is selected from lithium, sodium, potassium, rubidium, cesium, magnesium, calcium, strontium, barium, aluminium, tin, antimony, bismuth, zinc, cadmium, copper, manganese, chromium and vanadium and compounds of these metals.

8. The process as defined in Claim 4 or Claim 7 characterised in that said promoter is selected from oxides, hydroxides, halides, oxyhalides, hydrides, carbonyls, alkoxides, sulfates, sulfites, nitrates, nitrites, phosphates, phosphites, and organic carboxylates of metals.

9. The process as defined in any one of Claims 1 to 8 characterised in that said reaction is carried out in the presence of a solvent, said solvent being an organic material having a dielectric constant of not more than 18 at 25°C.

10. The process as defined in Claim 9 characterised in that the solvent is selected from aliphatic hydrocarbons, aromatic hydrocarbons, organic acid esters, ethers, ketones, alcohols, acid anhydrides and halogenated hydrocarbons.

11. The process as defined in Claim 10 characterised in that said solvent is ethylidene diacetate or acetic anhydride.

12. The process as defined in any one of Claims 1 to 11 characterised in that said reaction is carried out while removing vinyl acetate and acetic acid from the reaction system by distillation.

13. A process for continuously producing vinyl acetate which comprises reacting one or both of methyl acetate and dimethyl ether as a starting material with hydrogen and carbon monoxide in the presence of a catalyst and optionally a solvent and/or a promoter, characterised in that the catalyst comprises

(a) at least one material selected from metals belonging to Group VII of the Periodic Table, compounds of the metals and mixtures thereof and

(b) at least one iodide; and an apparatus for the reaction comprises a reaction zone and at least one separating zone and the process comprises

(i) continuously charging said starting material into the reaction zone,

(ii) continuously feeding carbon monoxide and hydrogen under pressure in the reaction zone, and

(iii) maintaining the concentration of the resultant vinyl acetate and acetic acid in the reaction system at less than 25% and less than 50% by weight, respectively, by removing part of the resulting vinyl acetate and acetic acid from the reaction system continuously.

**Patentansprüche**

1. Verfahren zur Herstellung von Vinylacetate, dadurch gekennzeichnet, daß man Methylacetat und/oder Dimethylether in Gegenwart eines Katalysators aus

a) mindestens einem Metall der Gruppe VIII des Periodensystems und/oder mindestens einer Verbindung eines solchen Metalls und

b) mindestens einem Jodid

mit Wasserstoff und Kohlenmonoxid umsetzt, wobei man durch Abziehen von Vinylacetat und Essigsäure aus dem Reaktionssystem die Konzentrationen an gebildetem Vinylacetat und gebildeter Essigsäure (im Reaktionssystem) auf unter 25 Gew.-% bzw. unter 50 Gew.-% hält.

2. Verfahren nach Anspruch 1, dadurch gekennzeichnet, daß man als Katalysatorkomponente (a) Palladium, Rhodium, Kobalt, Nickel oder eine Verbindung dieser Metalle verwendet.

3. Verfahren nach Anspruch 1 oder 2, dadurch gekennzeichnet, daß man als Jodid mindestens ein Alkyljodid, Säurejodid und/oder Jodwasserstoff verwendet.

4. Verfahren nach einem der Ansprüche 1 bis 3, dadurch gekennzeichnet, daß man die Umsetzung in Gegenwart des Katalysators und mindestens eines Beschleunigers, bestehend aus mindestens einer Verbindung der Stickstoffgruppe, einem ein Atomgewicht von mindestens 6 aufweisenden Metall der Gruppen Ia, IIa, IIIa, IVa, Va, Ib, IIb, Vb, VIb und VIIb des Periodensystems und/oder einer Verbindung dieser Metalle, durchführt.

5. Verfahren nach Anspruch 4, dadurch gekennzeichnet, daß man als Beschleuniger eine organische Verbindung mit dreiwertigem Phosphor oder dreiwertigem Stickstoff verwendet.

6. Verfahren nach Anspruch 4 oder 5, dadurch gekennzeichnet, daß man als Verbindung der Stickstoffgruppe eine heterocyclische Verbindung verwendet.

7. Verfahren nach Anspruch 4, dadurch gekennzeichnet, daß man als Beschleuniger Lithium, Natrium, Kalium, Rubidium, Cäsium, Magnesium, Calcium, Strontium, Barium, Aluminium, Zinn, Antimon, Wismuth, Zink, Cadmium, Kupfer, Mangan, Chrom und/oder Vanadium oder eine Verbindung dieser Metalle verwendet.

8. Verfahren nach Anspruch 4 oder 7, dadurch gekennzeichnet, daß man als Beschleuniger mindestens ein Metalloxid, -hydroxid, -halogenid, -oxyhalogenid, -hydrid, -carbonyl, -alkoxid, -sulfat. -sulfit, -nitrat, -nitrit, -phosphat, -phosphit oder organisches Metallcarboxylat verwendet.

9. Verfahren nach einem der Ansprüche 1 bis 8, dadurch gekennzeichnet, daß man die Umsetzung in Gegenwart eines organischen Lösungsmittels einer Dielektrizitätskonstante von höchstens 18 bei 25°C durchführt.

10. Verfahren nach Anspruch 9, dadurch gekennzeichnet, daß man als Lösungsmittel aliphatische Kohlenwasserstoffe, aromatischen Kohlenwasserstoffe, organische Säureester, Ether, Ketone, Alkohole, Säureanhydride und halogenierte Kohlenwasserstoffe verwendet.

11. Verfahren nach Anspruch 10, dadurch gekennzeichnet, daß man als Lösungsmittel Ethylidendiacetat oder Essigsäureanhydrid verwendet.

12. Verfahren nach einem der Ansprüche 1 bis 11, dadurch gekennzeichnet, daß man die Umsetzung unter Abdestillieren von Vinylacetat und Essigsäure aus dem Reaktionssystem durchführt.

13. Verfahren zur kontinuierlichen Herstellung von Vinylacetat durch Umsetzen von Methylacetat und/oder Dimethylether als Ausgangsmaterial mit Wasserstoff und Kohlenmonoxid in Gegenwart eines Katalysators und gegebenenfalls eines Lösungsmittels und/oder eines Beschleuigers, dadurch gekennzeichnet, daß man einen Katalysator aus

a) mindestens einem Metall der Gruppe VIII des Periodensystems und/oder mindestens einer Verbindung eines solchen Metalls und

b) mindestens einem Jodid

verwendet, wobei man in einer für die Umsetzung vorgesehen Vorrichtung mit einer Reaktionszone und mindestens einer Trennzone

(i) das Ausgangsmaterial kontinuierlich in die Reaktionszone einbringt,

(ii) Kohlenmonoxid und Wasserstoff kontinuierlich unter Druck in die Reaktionszone einführt und

(iii) die Konzentrationen an gebildetem Vinylacetat und gebildeter Essigsäure im Reaktionssystem durch kontinuierliches Abziehen eines Teils des Vinylacetats und der Essigsäure aus dem Reaktionssystem auf unter 25 Gew.-% bzw. unter 50 Gew.-% hält.

**Revendications**

1. Procédé de production d'acétate de vinyle, caractérisé en ce qu'il consiste à faire réagir de l'acétate de méthyle et du diméthyl éther ou les deux, avec de l'hydrogène et de l'oxyde de carbone en présence d'un catalyseur comprenant

(a) au moins un matériau choisi dans le groupe consistant en métaux appartenant au groupe VIII de la Table Périodique, composés des métaux et leurs mélanges et

(b) au moins un iodure, tout en maintenant les concentrations de l'acétate de vinyle et de l'acide acétique résultants dans le système réactionnel à moins de 25% et moins de 50% en poids, respectivement, en retirant l'acétate de vinyle et l'acide acétique du système réactionnel.

2. Procédé selon la revendication 1, caractérisé en ce que le composant (a) du catalyseur est choisi parmi le palladium, le rhodium, le cobalt, le nickel et des composés de ces métaux.

3. Procédé selon la revendication 1 ou 2, caractérisé en cde que l'iodure est choisi parmi es iodures d'alkyle, des iodures d'acide, de l'iodure d'hydrogène et leurs mélanges.

4. Procédé selon l'une quelconque des revendications 1 à 3, caractérisé en ce que ladite réaction est mise en oeuvre en présence dudit catalyseur et avec au moins un promoteur, ledit promoteur étant choisi parmi des composés du groupe azote, des métaux ayant un poids atomique d'au moins 6 et appartenant aux Groupes Ia, IIa, IIIa, IVa, Va, Ib, IIb, Vb, VIb et VIIb de la Table Périodique et composés de ces métaux.

5. Procédé selon la revendication 4, caractérisé en ce que ledit promoteur est un composé du groupe azote choisi parmi des composés organiques contenant du phosphore trivalent et des composés organiques contenant de l'azote trivalent.

6. Procédé selon la revendication 4 ou la revendication 5, caractérisé en ce que le composé du groupe azote est un composé hétérocyclique.

7. Procédé selon la revendication 4, caractérisé en ce que ledit promoteur est choisi parmi le lithium, le sodium, le potassium, le rubidium, le césium, le magnésium, le calcium, le strontium, le baryum, l'aluminium, l'étain, l'antimoine, le bismuth, le zinc, le cadmium, le cuivre, le manganèse, le chrome et le vanadium et des composés de ces métaux.

8. Procédé selon la revendication 4 ou la revendication 7, caractérisé en que ledit promoteur est choisi parmi des oxydes, hydroxydes, halogénures, oxyhalogénures, hydrures, carbonyles, alcoolates, sulfates, sulfites, nitrates, phosphates, phosphites et carboxylates organiques de métaux.

9. Procédé selon l'une quelconque des revendications 1 à 8, caractérisé en ce que ladite réaction est mise en oeuvre en présence d'un solvant, ledit solvant étant un matériau organique ayant une constante diélectrique de pas plus de 18 à 25°C.

10. Procédé selon la revendication 9, caractérisé en ce que le solvant est choisi parmi des hydrocarbures aliphatiques, des hydrocarbures aromatiques, des esters d'acides organiques, des éthers, des cétones, des alcools, des anhydrides d'acides et des hydrocarbures halogénés.

11. Procédé selon la revendication 10, caractérisé en ce que ledit solvant est du diacétate d'éthylidène ou de l'anhydride acétique.

12. Procédé selon l'une quelconque des revendications 1 à 11, caractérisé en ce que ladite réaction est mise en oeuvre tandis que l'on retire l'acétate de vinyle et l'acide acétique du système réactionnel, par distillation.

13. Procédé pour produire continuellement de l'acétate de vinyle qui consiste à faire réagir de l'acétate de méthyle et du diméthyl éther ou les deux comme matière première avec de l'hydrogène et de l'oxyde de carbone en présence d'un catalyseur et éventuellement d'un solvant et/ou d'un promoteur, caractérisé en ce que le catalyseur comprend

(a) au moins un matériau choisi parmi des métaux appartenant au Groupe VIII de la Table Périodique, des composés des métaux et leurs mélanges et

(b) au moins un iodure; et un dispositif pour la réaction comprend une zone de réaction et au moins une zone de séparation et le procédé consiste à

(i) introduire continuellement ladite première dans la zone de réaction,

(ii) amener continuellement l'oxyde de carbone et l'hydrogène sous pression dans la zone de réaction, et

(iii) maintenir les concentrations de l'acétate de vinyle et de l'acide acétique résultants dans le système réactionnel à moins de 25% et moins de 50% en poids, respectivement, en retirant continuellement une partie de l'acétate de vinyle et de l'acide acétique résultants, du système réactionnel.

Fig. 1

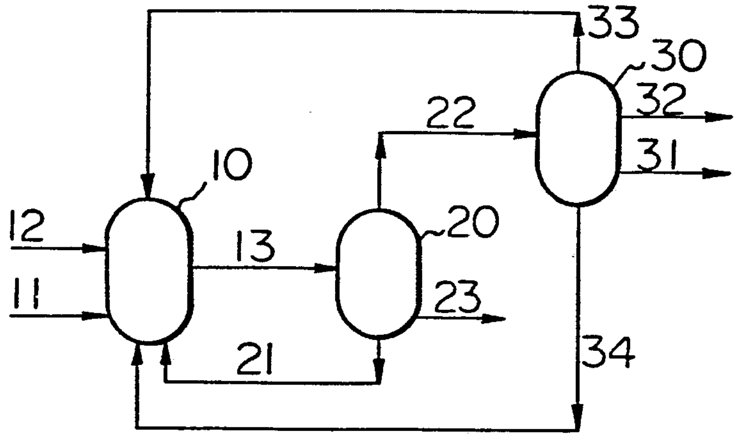
33
30
32
22
10
31
12
20
13
11
23
21
34

Fig. 2

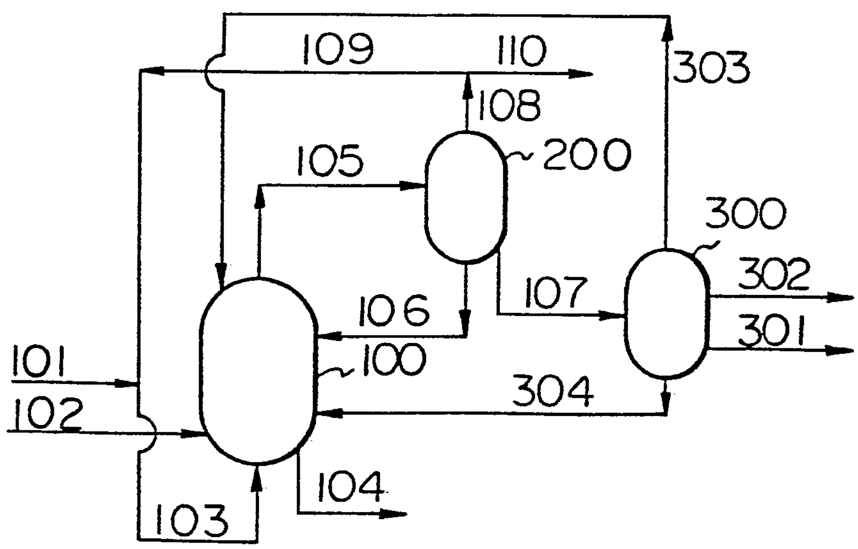
109
110
303
108
105
200
300
302
106
107
301
101
100
304
102
104
103